# EUROPEAN PATENT SPECIFICATION

(11) **EP 0 359 516 B1**
(45) Date of publication and mention of the grant of the patent: **10.08.1994**
(21) Application number: 89309235.3
(22) Date of filing: 12.09.1989
(51) Int. Cl.: C07D 263/08

(54) **Process for preparing oxazoline compounds**
Verfahren zur Herstellung von Oxazolinderivaten
Procédé de préparation d'oxazolines

(30) Priority: 14.09.1988 US 244126
(43) Date of publication of application: 21.03.1990
(73) Proprietor: SCHERING CORPORATION, Kenilworth New Jersey 07033 (US)
(72) Inventor: Schumacher, Doris P., Florham Park New Jersey 07932 (US); Clark, Jon E., Highland Park New Jersey 08904 (US); Murphy, Bruce L., Glen Ridge New Jersey 07028 (US)
(74) Representative: Ritter, Stephen David

(56) References cited:
- EP-A- 0 123 123
- US-A- 2 402 198
- US-A- 3 979 405
- JUSTUS LIEBIGS ANNALEN DER CHEMIE, no. 6, 6th June 1974, pages 996-1009, Weinheim, DE; H. WITTE et al.: "Cyclische Imidsäureester aus Nitrilen und Aminoalkoholen"

## Description

### FIELD OF THE INVENTION

The present invention is directed to a novel process for preparing oxazoline compounds. Oxazoline compounds are useful in preparing surface active agents, detergents, waxes, and intermediates for pharmaceutical compounds, such as those disclosed in U.S. Patent 4,743,700.

### BACKGROUND OF THE INVENTION

Oxazoline compounds are known, and are disclosed in U.S. Patents 4,216,162; 3,813,378; 2,786,870; 2,718,520; and 2,820,041.

Processes for making oxazolines are known, such as described in European Patent Application 130,633, U.S. Patent 4,235,892 and the article "Formation of Cyclic Imidic Esters by Reaction of Nitriles with Amino Alcohols" by H. Witte and Wolfgang Seeliger, Liebigs Ann Chem. pp 996-1009, (1974) which utilizes catalytic amounts of certain metal salts. U.S. Patent 2,402,198 describes a process for preparing oxazolines by reacting monoethanolamine with a nitrile in the presence of an alkaline catalyst. U.S. Patent 2,759,001 discloses preparing racemic mixtures of isomeric oxazolines by reacting dichloroacetonitrile with an aminodiol compound. U.S. Patent 3,979,405 discloses preparing 2-oxazolines by reacting an amino alcohol with a nitrile in an anhydrous alcohol such as n-butanol or cyclohexanol. None of these references teach a method for preparing oxazolines employing a dihydric or polyhydric alcohol solvent. It would be desirable to provide a process for preparing oxazoline compounds whose yields, purity and selectivity are as good as or better than methods previously taught. It would also be desirable to provide a process for preparing said oxazoline compounds which requires as few or even fewer steps than methods previously taught. It would also be desirable to provide a process which is as economical, if not more so, than previous methods. Further, where two or more potential structural isomers may be formed, it would be highly desirable to provide a process which is regio-selective i.e. produces only one structural isomer.

### SUMMARY OF THE INVENTION

The present invention is directed toward a process for preparing oxazoline compounds of the formula: 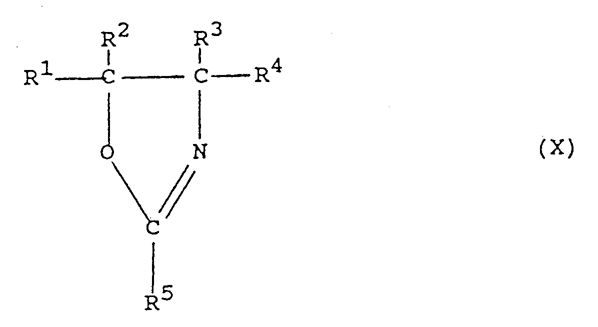
Wherein R¹,R²,R³,R⁴ and R⁵ can be the same or different, provided that R⁵ does not contain cyano or carboxyl groups, each independently represents
hydrogen, alkyl, haloalkyl, cycloalkyl or substituted cycloalkyl, cycloalkalkyl or substituted cycloalkalkyl, alkenyl or substituted alkenyl, alkynyl, alkenylalkyl or substituted alkenylalkyl, alkynylalkyl, alkoxyalkyl or substituted alkoxyalkyl, dialkylaminoalkyl, aryl or substituted aryl, arylalkyl or substituted arylalkyl, arylalkenyl or substituted arylalkenyl, alkoxyaryl or substituted alkoxyaryl, aryloxyaryl or substituted aryloxyaryl, aryloxyalkyl or substituted aryloxyalkyl, acyl or substituted acyl, aromatic heterocyclic or substituted aromatic heterocyclic, heterocyclic alkyl or substituted heterocyclic alkyl, heterocyclic cycloalkyl or substituted heterocyclic cycloalkyl, heterocyclic cycloalkyalkyl or substituted heterocyclic cycloalkylalkyl, sulfoxide or substituted sulfoxide, sulfonyl or substituted sulfonyl, sulfide or substituted sulfide or hydroxyalkyl.

The process comprises the step of contacting a cyano compound of the formula

R⁵ - C≡N (VIII)

with an aminoalcohol compound of the formula 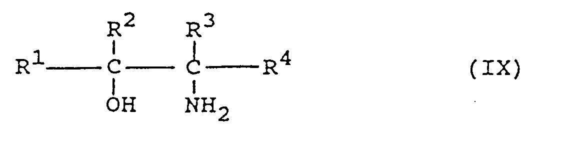
or salt thereof, wherein R¹, R², R³, R⁴ and R⁵ are as defined hereinbefore, performed in the presence of a base and an alcohol solvent selected from ethylene glycol, glycerol or mixtures thereof.

In a preferred embodiment, R¹ and R² independently represent hydrogen, sulfide, sulfoxide, sulfonyl, aryl, or substituted aryl and R³ and R⁴ independently represent hydrogen or -CH₂OH.

In a further preferred embodiment R¹ and R² independently represent hydrogen or 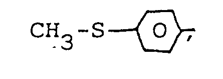 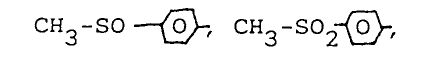
and R³ and R⁴ independently represent hydrogen or -CH₂OH.

In a further preferred embodiment R³ and R⁴ independently represent alkyl or hydroxyalkyl.

R⁵ is preferably phenyl, 4-nitrophenyl, cynnamyl, 4-methoxy cynnamyl or dichloromethyl.

More preferably, R² is 4-methylthiophenyl, 4-methyl-SO-phenyl or 4-methyl-SO₂-phenyl; R⁴ is hydroxymethyl and R⁵ is phenyl or dichloromethyl when R¹ and R³ are hydrogen. Preferably the base is diazabicycloundecene, more specifically 1,8-diazobicyclo[5.4.0]undec-7-ene (DBU) or an alkali metal carbonate, most preferably potassium carbonate. Also, preferred is that the alcohol solvent is a mixture of ethylene glycol and glycerol.

Also preferred are those amino alcohol starting materials, (IX) of the general formula 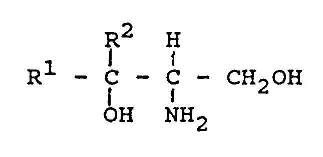
wherein R¹ and R² are as defined hereinabove. For these starting materials the process is regio-selective. That is, although the reaction potentially can give rise to two or more structural isomers, the present process produces only one of the two or more potential structural isomers.

The process has the advantages of being able to prepare an oxazoline compound of formula (X) in high yields, good purity, high specificity, with low by-product formation using relatively mild reaction conditions with as few or fewer steps than other processes previously taught. The present invention has the further advantage of providing a process of preparing oxazoline compounds as economically, if not more so, than other processes previously taught. The present invention has the further advantage of providing a process for preparing oxazoline compounds whose stereoisomeric configuration can be easily determined aforehand simply by the selection of the appropriate starting materials. An especially advantageous feature of the present invention is that virtually no racemization occurs when chiral starting material of formula IX are used. In the particular situation where the amino alcohol starting material contains two hydroxyl groups, the present process has the advantage in that it is regio-selective.

### DETAILED DESCRIPTION OF THE EMBODIMENTS

When utilized in the present specification and in the appended claims the terms listed hereinbelow, unless otherwise indicated are defined as follows:

The term "alkyl" refers to a straight saturated hydrocarbon moiety (i.e. hydrocarbons having carbon-carbon single bonds) containing from 1 to 6 carbon atoms, or a branched saturated hydrocarbon moiety of 3 to 6 carbon atoms, such as for example, methyl (i.e. -CH₃), ethyl, propyl, isopropyl, butyl, isobutyl, tert-butyl, pentyl, hexyl and the like;
the term "substituted" refers to a moiety which is further substituted by one or more of the following groups: halo, alkyl of one to six carbon atoms, aryl of six to fifteen carbon atoms, cyano (i.e. -CN), carboxyl (i.e. -COOH) or salts thereof, nitro (i.e. -NO₂) and hydroxyl;
The terms "halogen" and "halo" refers to fluoride, chloride, bromide or iodide.

The term "haloalkyl" refers to an alkyl moiety in which one or more of the hydrogen atoms has been replaced by a halogen atom, such as, for example, chloromethyl, fluoromethyl, bromomethyl, trifluoromethyl, dichloromethyl, 2-chloro-2-fluoroethyl, 6,6,6-trichlorohexyl and the like.

The term "cycloalkyl" refers to a saturated carbocyclic ring characterized by closed rings and containing from 3 to 6 carbon atoms, such as for example, cyclopropyl, cyclobutyl, cyclopentyl, cyclohexyl, and the like;
The term "cycloalkalkyl" refers to a cycloalkyl moiety of 3 to 6 carbon atoms covalently bonded to an alkyl moiety of 1 to 6 carbon atoms;
The term "alkenyl" refers to a straight hydrocarbon moiety of two to six carbon atoms or a branched hydrocarbon moiety of three to six carbon atoms having at least one carbon-carbon double bond, such as ethenyl (i.e. -CH=CH₂), propenyl, 1-butenyl, 2-butenyl, isobutenyl, 1-pentenyl, 2-methyl-1-butenyl, 1-hexenyl and the like;
The term "alkynyl" refers to a straight hydrocarbon moiety of two to six carbon atoms or a branched hydrocarbon moiety of four to six carbon atoms having one carbon to carbon triple bond such as ethynyl (i.e. -C≡CH), 1-propynyl, 1-butynyl, 1-pentynyl, 2-pentynyl, 1-hexynyl, 2-hexynyl, 3-hexynyl and the like;
The term "alkoxy" refers to an alkyl moiety containing from 1 to 6 carbon atoms covalently bonded to an adjacent structural element through an oxygen atom, such as for example, methoxy (i.e. -OCH₃), ethoxy, propoxy, isopropoxy, butoxy, pentoxy, hexoxy and the like;
The term "alkenylalkyl" refers to an alkenyl moiety of two to six carbon atoms covalently bonded to a alkyl moiety of 1 to 6 carbon atoms;
The term "alkynylalkyl" refers to an alkynyl moiety of two to six carbon atoms covalently bonded to an alkyl moiety of 1 to 6 carbon atoms;
The term "alkoxyalkyl" refers to an alkoxy moiety of 1 to 6 carbon atoms covalently bonded to an alkyl moiety of 1 to 6 carbon atoms;
The term "amino" refers to a primary (-NH₂), a secondary or a tertiary amine wherein each hydrogen can be substituted by an alkyl moiety of one to six carbon atoms or by an aryl moiety of six to fifteen carbon atoms.

The term "dialkylaminoalkyl" refers to a nitrogen atom covalently bonded to three alkyl moieties having 1 to 6 carbon atoms in each alkyl moiety, and one alkyl moiety is bonded to an adjacent structural element.

The term "aryl" refers to a carbocyclic moiety containing at least one benzenoid-type ring, with the aryl groups preferably containing from 6 to 15 carbon atoms, for example, phenyl, naphthyl, indenyl, indanyl, and the like;
The term "arylalkyl" refers to an aryl moiety of 6 to 15 carbon atoms covalently bonded to an alkyl moiety of one to six carbon atoms such as, for example, benzyl, phenylethyl, and the like;
The term "arylalkenyl" refers to an aryl moiety of six to fifteen carbon atoms covalently bonded to an alkenyl moiety of two to six carbon atoms, such as, for example, 2-phenyl-1-ethenyl (cinnamyl), 4-phenyl-2-butenyl and the like;
The term "aryloxy" refers to an aryl moiety covalently bonded through an oxygen (i.e. -O-) atom, such as, for example, phenoxy and the like;
The term "alkoxyaryl" refers to an alkoxy moiety of one to six carbon atoms covalently bonded to an aryl moiety of six to 15 carbon atoms, such as, for example 2-methoxyphenyl, 2- or 4-ethoxynaphthyl, 6-propoxyindenyl and the like;
The term "aryloxyaryl" refers to an aryloxy moiety as defined hereinbefore covalently bonded to an aryl moiety of six to 15 carbon atoms, such as, for example, phenoxyphenyl, 1-naphthyloxyphenyl and the like;
The term "aryloxyalkyl" refers to an aryloxy moiety as defined hereinbefore covalently bonded to an alkyl moiety of one to six carbon atoms, such as, for example, phenoxymethyl, 1-naphthyloxyethyl and the like;
The term "acyl" refers to a carbonyl moiety 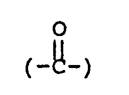
bonded to a hydrogen, alkyl, aryl, alkoxy, amino or an aryloxy group such as a formyl moiety 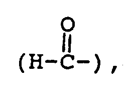
an alkanoyl moiety 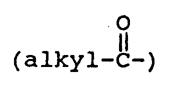
of one to six carbon atoms in the alkyl portion, an aroyl moiety 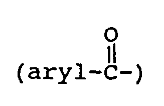
of six to 15 carbon atoms in the aryl portion, an ester moiety 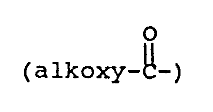
of one to six carbon atoms in the alkoxy portion, an amide moiety 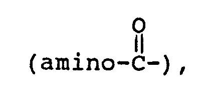
or an aryloxy moiety. Typical acyl groups include acetyl, benzoyl, ethoxycarbonyl and the like;
The term "aromatic heterocyclic" refers to a cyclic moiety having at least one O, S and/or N heteroatom interrupting the ring structure and having a sufficient number of unsaturated carbon to carbon double bonds, nitrogen to carbon double bonds, and the like, to provide aromatic character, with the aromatic heterocyclic groups preferably containing from 2 to 14 carbon atoms, for example, 2-, 3- or 4-pyridyl, 2- or 3-furyl, 2- or 3-thienyl, 2-, 4- or 5-thiazolyl, 2-, 4- or 5-imidazolyl, 2-, 4- or 5-pyrimidinyl, 2-pyrazinyl, 3- or 4-pyridazinyl, 3-, 5- or 6-[1,2,4-triazinyl], 3- or 5-[1,2,4-thiadiazolyl], 2-, 3-, 4-, 5-, 6- or 7-benzofuranyl, 2-, 3-, 4-, 5-, 6- or 7-indolyl, 3-, 4- or 5-pyrazolyl, 2-, 4- or 5-oxazolyl, and the like;
The term "heterocyclic alkyl" refers to an aromatic heterocyclic moiety of 2 to 14 carbon atoms as defined hereinbefore, covalently bonded to an alkyl moiety of one to six carbon atoms;
The term "heterocyclic cycloalkyl" refers to a saturated carbocyclic ring of two to six carbon atoms having at least one oxygen, sulfur or nitrogen atom, or combination thereof, interrupting the ring structure, and are substituted at a carbon atom, such as 2-, 3-, or 4-piperidyl, 2-dioxanyl, 2- or 3-oxazetidinyl, 2-oxiranyl, or 3-, 4-, 5- or 6-thiazinyl and the like;
The term "heterocyclic cycloalkylalkyl" refers to a heterocyclic cycloalkyl moiety of two to six carbon atoms covalently bonded to an alkyl moiety of one to six carbon atoms;
The term "sulfoxide" refers to a sulfoxide moiety (i.e. R-SO-R-) wherein each R independently represents an alkyl moiety of one to six carbon atoms or an aryl moiety of 6 to 15 carbon atoms, such as, for example, 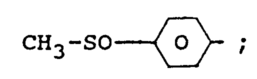
The term "sulfonyl" refers to a sulfonyl moiety (i.e. R-SO₂-R-) wherein each R independently represents alkyl of one to six carbon atoms or aryl of six to twelve carbon atoms, such as, for example, 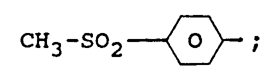
The term "sulfide" refers to a sulfide moiety (i.e. R-S-R-) wherein each R independently represents alkyl of one to six carbon atoms or aryl of six to twelve carbon atoms, such as, for example, alkylthioalkyl, or alkylthioaryl such as 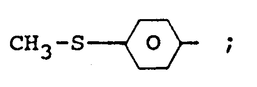
The term "hydroxyalkyl" refers to an alkyl moiety in which one or more of the hydrogens is replaced by a hydroxy moiety, such as, for example, hydroxymethyl (i.e. -CH₂OH), hydroxyethyl, 1-hydroxypropyl, 2-hydroxypropyl, 3-hydroxybutyl, 4-hydroxyhexyl and the like.

The base employed in the present process is any substance which will remove a proton from the hydroxyl (-OH) of the moiety 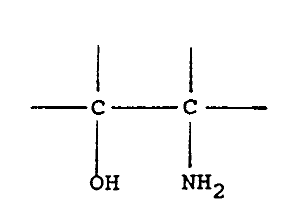
in order to cyclize the amino alcohol (IX) with the cyano compound (VIII) to give the desired oxazoline compound (X). The base is neither the amino alcohol (IX) nor the cyano compound (VIII).

Bases which can be employed in process of the present invention can be a non-aqueous base such as lithium diisopropyl amide, lithium hexamethylsilazide, sodium hexamethylsilazide and potassium hexamethylsilazide; or potassium t-butoxide and sodium methoxide. The base can be an alkali metal carbonate such as sodium, potassium, lithium or cesium carbonate or an alkaline earth metal carbonate such as calcium or barium carbonate; hydroxides such as sodium and potassium hydroxides; and hydrides such as sodium or potassium hydrides. The base can also be ammonia (NH₃) or an organic base including urea; a secondary amine such as dimethylamine, diphenylamine, N-methyl N-propylamine, diethylamine, diisopropylamine, N-methylaniline, piperazine, piperidine, pyrrolidine; or a tertiary amine such as trimethylamine, dimethylaniline, N,N-dimethylpropylamine, N,N-dimethylpiperidine, N,N-dimethylbutylamine, triethylamine. The base can also be an heterocyclic nitrogen containing compound such as isoquinoline, morpholine, purine, pyridine, pyrazine, pyrimidine, quinoline or polyvinyl pyridine, preferably DBU. Other bases which may be suitably employed in the present process are disclosed in "Modern Synthetic Reactions" by H. House, W.A. Benjamin, Inc., Menlo Park, California, 1972, 856 pages. Where appropriate, mixtures of any of the above bases can be employed.

The base is used in amounts effective to remove the requisite proton from the hydroxy moiety. Such amounts in terms of mole ratios (moles base:mole amino alcohol (IX)) can range from about 1,000 to 0.001:1, preferably from about 10 to 0.01:1, more preferably from about 1 to 0.1:1, most preferably about 0.15:1.

The alcohol solvent employed in the present invention is selected from ethylene glycol, glycerol and mixtures thereof.

Where suitable, the process of the present invention can employ a mixture of ethylene glycol and glycerol in a volume ratio of 1:2 (ethylene glycol:glycerol).

The alcohol solvent employed in the present process can be used in amounts which can range from an amount sufficient to at least partially solubilize one or both of the reactants and/or the desired product to an amount in excess of either starting reactant. Generally the amount of alcohol solvent can range from about 1 to 5,000 percent or more by weight of an individual reactant, preferably from about 100 to 1,000 percent by weight, most preferably from about 100 to about 300 percent.

In the process of preparing the oxazoline compound of formula (X), the cyano compound (VIII) is contacted with the aminoalcohol (IX) in amounts and under conditions effective to yield the desired oxazoline compound of formula (X). The cyano compound (VIII) is contacted with the aminoalcohol (IX) at temperatures ranging from about -10 to about 200 degrees Centigrade (°C), preferably from about 70 to about 150°C, most preferably from about 100 to about 110°C. The contacting is performed at ambient pressures although pressures greater or less than ambient can be employed. The contacting of the reactants can be carried out for about 5 minutes to about 72 hours or more until the reaction is substantially completed, preferably from about 1 hour (hr) to about 48 hours. Also preferred is that the reactants are stirred during the contacting procedures. The cyano compound (VIII) can be contacted with the amino alcohol of (IX) in molar ratios ranging from about 100 to 0.1:1; (moles cyano compound (VIII): mole amino alcohol (IX)), preferably from about 10 to 1:1, most preferably from about 2 to 1:1.

The stereochemistry of the oxazoline compounds (X) is preserved with respect to the stereochemistry of the starting materials. For example, when an amino alcohol of S,S′ configuration is contacted with benzonitrile, the resultant oxazoline (X) has a S,S′ stereoisomeric configuration.

After the reaction is completed, the desired oxazoline compound (X) can be recovered by conventional separatory and recovery methods such as phase separation, distillation or evaporation of any solvents present, crystallization, chromatography, filtration and the like. For example, the reaction mixture can be diluted with water and the oxazoline compound (X) is recovered by filtration.

### Preparation of Starting Materials

The cyano compounds (VIII) are known and can be prepared by conventional procedures, such as for example, by dehydration of the corresponding amide with a dehydrating agent such as phosphorous oxychloride. The dehydration reaction is illustrated as follows: 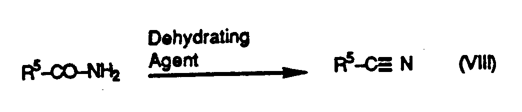
wherein R⁵ is as defined hereinbefore. Similarly, other methods for preparing the cyano compounds (VIII) are disclosed in references such as H.O. House, Modern Synthetic Reactions, Second Edition, W.A. Benjamin Inc., (1979) pp. 79 and 623-628, whose preparation teachings are incorporated herein by reference.

The aminoalcohol compounds (IX) are known and can be prepared by conventional procedures, such as, for example by epoxidation of the corresponding olefin (V) by an epoxidizing method, followed by cleavage of the epoxide ring (VI) by azide to give the azido compound (VII), followed by reduction of the azido compound with a reducing agent to give the requisite aminoalcohol (IX). The procedure can be illustrated as follows: 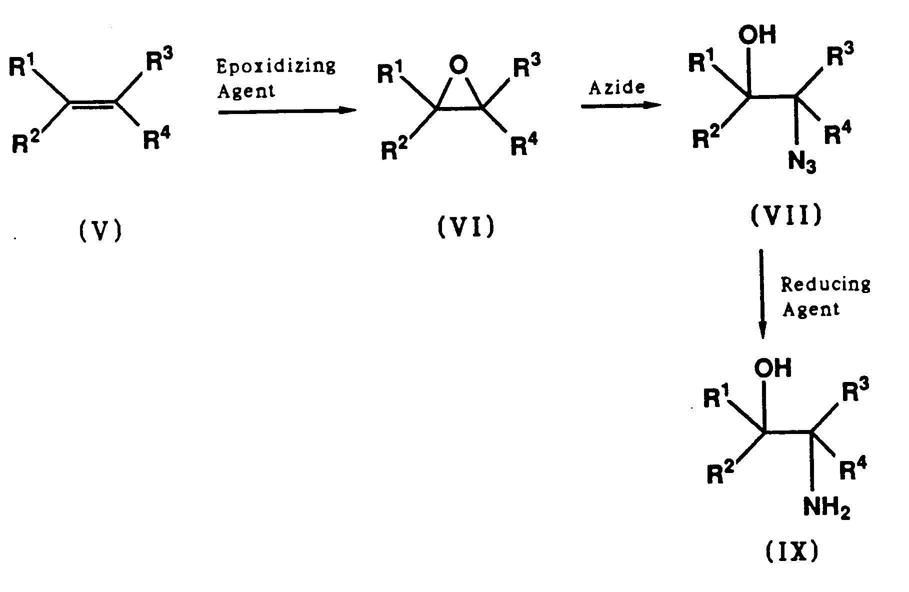
wherein R¹, R², R³ and R⁴ are as defined hereinbefore. Representative epoxidizing methods include any suitable peracid reactant such as, for example, pertrifluoroacetic acid (CF₃COOH ), perbenzoic acid (C₆H₅COOH ), peracetic acid (CH₃COOH ) and the like, as well as formation of a halohydrin followed by base.

In situations where any of R¹, R², R³ or R⁴ of olefin compound (V) contains an oxidizable functionality, such as an unsaturated bond, the olefin compound (V) can be selectively epoxidized and/or the requisite epoxide ring compound (VI) thus prepared can be further isolated in order to prepare the azido compound (VII). Representative azides include those of alkali earth metals, such as sodium azide, potassium azide, lithium azide, and the like.

The term "reducing agent" refers to any substance which will furnish electrons by its capacity to lose electrons easily, in order to cause the azido compound (VII) receiving the electrons to be reduced to the desired amino alcohol (IX). Examples of reducing agents include, but are not limited to hydrogenating agents and to metal hydrides, such as lithium aluminum hydride (LiAlH₄).

The term "hydrogenating agent" is intended to include the requisite hydrogenating catalyst(s) and hydrogen (H₂) source for reducing the azido compound (VII) to the aminoalcohol (IX). Various selected catalysts and conditions are described in "Catalytic Hydrogenation in Organic Synthesis", (1978) Morris Freifelder, Chapter 4, Olefins, pg. 15-25, John Wiley and Sons. For example, the hydrogenating catalyst can be nickel, palladium, platinum, platinum oxide, platinum on carbon, and mixture thereof.

Similarly, in situations where R¹, R², R³ or R⁴ of azido compound (VII) contains a reducible functionality such as an unsaturated bond or a sulfur atom, azido compound (VII) can be reduced with a selective reducing agent and/or the requisite amino alcohol (IX) thus prepared can be further isolated in order to prepare oxazoline compound (X).

Other representative methods of preparing the aminoalcohols of formula (IX) are described in Leroy G. Wade, Jr., Vol. 5, John Wiley and Sons, (1984) pp. 430-431 and in Calvin Buchler and Donald Pearson, Survey of Organic Synthesis, Vol. 1, Wiley Interscience, New York (1970), pp. 226, 466 and 475. The preparative teachings of these references are incorporated herein by reference.

Salts of the aminoalcohol (IX) can be prepared by contacting the aminoalcohol (IX) with organic or inorganic acids. Representative organic acids include but are not limited to oxalic, tartaric, acetic, trifluoroacetic, citric, maleic and the like. Representative inorganic acids include hydrochloric, sulfuric or phosphoric acids in about equimolar amounts or in amounts less than equimolar of the acid relative to the aminoalcohol.

The following examples illustrate the present invention in a manner of which it can be practiced but, as such, should not be construed as limitations upon the overall scope of the same.

### EXAMPLE 1

### PREPARATION OF D-2-PHENYL-5-(4-METHYLMERCAPTOPHENYL)-4-HYDROXYMETHYL-2-OXAZOLINE

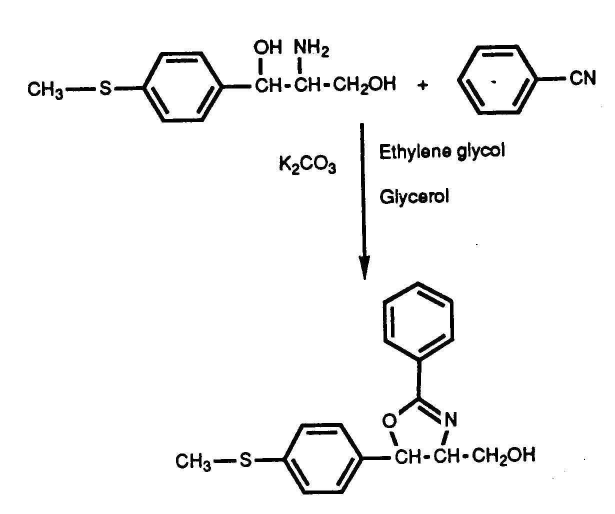

A suspension of 5 grams (g) (23.5 millimole (mmole)) of D(-)-2-amino-1-(4-methylmercaptophenyl)-1,3-propanediol (available from Fuji Chemical Co., Japan) and 0.5 g (3.6 mmole) of potassium carbonate (K₂CO₃) in 7.5 milliliters (mL) of ethylene glycol and 4.1 mL of glycerol is heated to a temperature of 105°C with stirring. Benzonitrile (4 mL, 39.2 mmole, 1.67 equivalents) is added and the mixture is stirred at 105°C for 18 hr under a blanket of nitrogen gas. Completeness of reaction is determined by thin layer chromatography (TLC) on silica gel (9:1 - methylene chloride: methanol). The product is isolated by precipitation into water, to give 6.8g (purity 97%, 22.0 mmole) a 93 percent yield.

### EXAMPLE 2

### PREPARATION OF 4-METHYL-2-PHENYL-2-OXAZOLINE

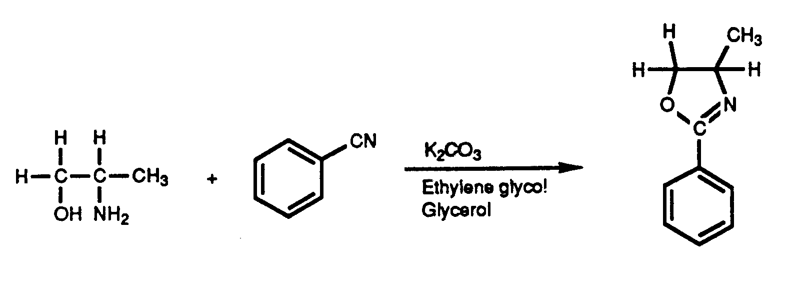

To a mixture of five mL of 2-aminopropanol (62.8 mmole) and 273 milligrams (mg) of potassium carbonate (37.7 mmole) in ethylene glycol (20.5 mL) and glycerol (11.5 mL) heated to a temperature of 105 degrees °C, is added benzonitrile (9.8 mL; 96.4 mmol)). The reaction mixture is stirred at 105°C for 18 hours, diluted with hexane and washed with water. The solvent and excess benzonitrile from the organic layer are removed by distillation to give 10.45g (purity 85 percent (%)), of title compound, a yield of 92 percent.

### EXAMPLE 3

### PREPARATION OF D-2-(p-NITROPHENYL)-5-(4-METHYLMERCAPTOPHENYL)-4-HYDROXYMETHYL -2-OXAZOLINE

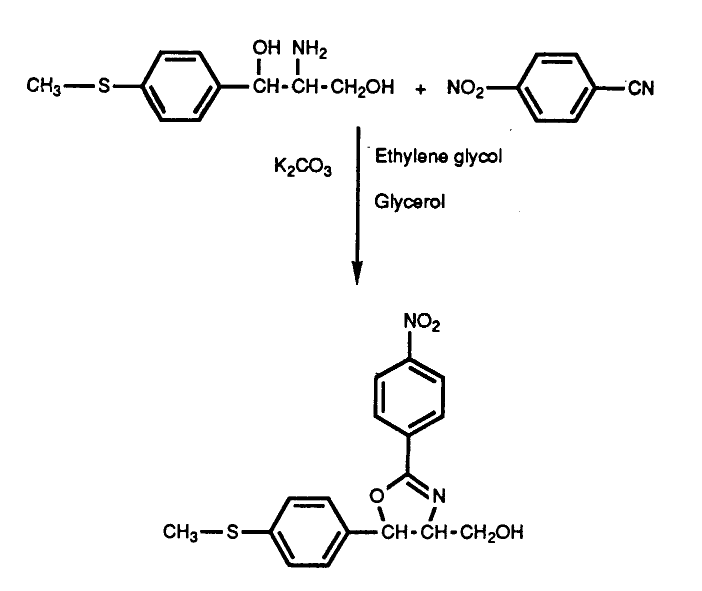

To a mixture of 1 g of D(-)-2-amino-1-(4-methylmercaptophenyl)-1,3-propanediol (4.7 mmole) and 0.1g of potassium carbonate in 1.5 mL of ethylene glycol and 0.8 mL of glycerol, heated to a temperature of 100°C, 1.0 g of benzonitrile is added, and stirred for 3 hours. Analysis of the reaction mixture by high pressure liquid chromatography indicates a yield of title compound to be 85 percent.

## Claims

1. A process for preparing an oxazoline compound of the formula: 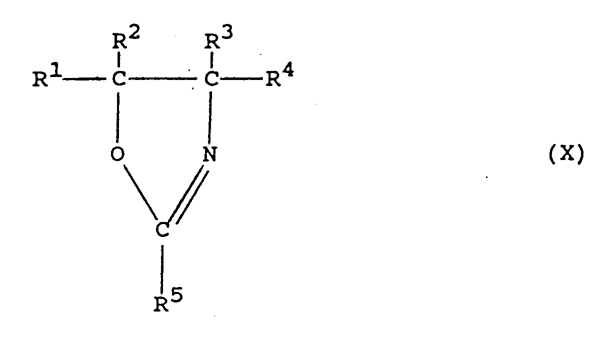 comprising contacting a cyano compound of the formula
R⁵ - C≡N (VIII)
with an amino alcohol compound of the formula 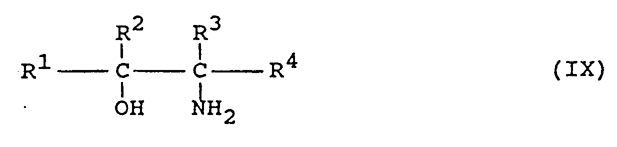 or salt thereof, in the presence of a base,
wherein R¹, R², R³, R⁴, and R⁵ can be the same or different, provided that R⁵ does not contain cyano or carboxyl groups, and each independently represents:-
(a) hydrogen, alkyl, alkynyl, alkynylalkyl, hydroxyalkyl, haloalkyl, dialkylaminoalkyl,
(b) cycloalkyl, cycloalkalkyl, heterocyclic alkyl, heterocyclic cycloalkyl, heterocyclic cycloalkalkyl, alkenyl, alkenylalkyl, alkoxyalkyl, aryl, arylalkyl, arylalkenyl, alkoxyaryl, aryloxyaryl, aryloxyalkyl, acyl, aromatic heterocyclic, sulfoxide, sulfonyl, sulfide, or
(c) the moieties listed at (b) substituted by halo, alkyl, aryl, cyano, carboxyl or salts thereof, nitro or hydroxyl,
wherein alkyl = C₁-C₆ alkyl,
aryl = C₆-C₁₅ aryl,
acyl= a carbonyl moiety bonded to hydrogen, alkyl, aryl, alkoxy, amino or aryloxy,
sulfoxide= R₆-SO-R₇- wherein R⁶ and R₇ are independently alkyl or aryl,
sulfonyl =R⁸-SO₂-R⁹, wherein R⁸ and R⁹ are independently alkyl or C₆-C₁₂ aryl,
sulfide =R⁸-S-R⁹, wherein R⁸ and R⁹ are as above;
characterised in that the reaction is performed using, as solvent, ethylene glycol, glycerol or mixtures thereof.

2. The process of claim 1 wherein as to the amino alcohol (IX), R¹ and R² independently represent hydrogen, sulfide, sulfoxide, sulfonyl, aryl or substituted aryl; and R³ and R⁴ independently represent hydrogen or -CH₂OH.

3. The process of claims 1 or 2 wherein R¹ and R² independently represent hydrogen or 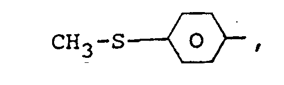 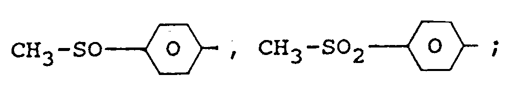 and R³ and R⁴ independently represent hydrogen or -CH₂OH.

4. The process of claims 1, 2 or 3 wherein as to the cyano compound (VIII) R⁵ is phenyl, 4-nitrophenyl, cinnamyl, 4-methoxycinnamyl or dichloromethyl.

5. The process of claims 1, 2, 3 or 4 wherein the base is diazabicycloundecene or potassium carbonate.

6. The process of claims 1, 2, 3, 4 or 5 wherein the base is employed in an amount ranging from about 10 to 0.01 moles base to one mole amino alcohol.

7. The process of claims 1, 2, 3, 4, 5 or 6 wherein the alcohol solvent is ethylene glycol.

8. The process of claims 1, 2, 3, 4, 5, 6 or 7 wherein the alcohol solvent is a mixture of ethylene glycol and glycerol.

9. The process of claims 1, 2, 3, 4, 5, 6, 7 or 8 wherein the contacting is performed at temperatures ranging from about 70 to about 150°C.

10. The process of claims 1, 2, 3, 4, 5, 6, 7 or 8 wherein the contacting is performed at temperatures ranging from about 100 to about 110°C.

## Patentansprüche

1. Verfahren zum Herstellen einer Oxazolinverbindung der Formel 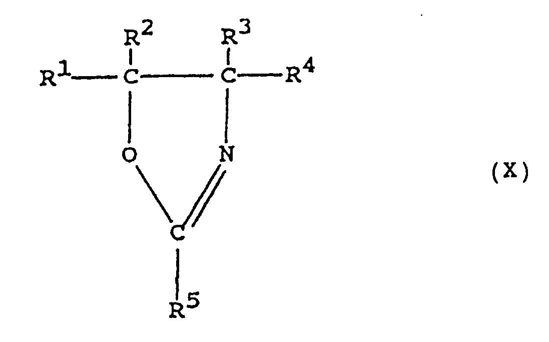 welches das Zusammenbringen einer Cyanverbindung der Formel
R⁵-C≡N (VIII)
mit einer Aminoalkoholverbindung der Formel 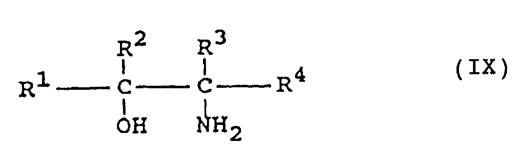 oder einem Salz derselben in Anwesenheit einer Base umfaßt, worin R¹, R², R³, R⁴ und R⁵ gleich oder verschieden sein können, vorausgesetzt, daß R⁵ keine Cyan- oder Carboxylgruppen enthält, und jedes unabhängig
(a) Wasserstoff, Alkyl, Alkinyl, Alkinylalkyl, Hydroxyalkyl, Halogenalkyl, Dialkylaminoalkyl,
(b) Cycloalkyl, Cycloalkalkyl, heterocyclisches Alkyl, heterocyclisches Cycloalkyl, heterocyclisches Cycloalkalkyl,Alkenyl, Alkenylalkyl, Alkoxyalkyl, Aryl, Arylalkyl, Arylalkenyl, Alkoxyaryl, Aryloxyaryl, Aryloxyalkyl, Acyl, aromatisches Heterocyclyl, Sulfoxid, Sulfonyl, Sulfid oder
(c) die in (b) aufgezählten, durch Halogen, Alkyl, Aryl, Cyan, Carboxyl oder deren Salze, Nitro oder Hydroxyl substituierten Struktureinheiten darstellt,
worin
Alkyl = C₁-C₆-Alkyl,
Aryl = C₆-C₁₅-Aryl,
Acyl = eine an Wasserstoff, Alkyl, Aryl, Alkoxy, Amino oder Aryloxy gebundene Carbonylstruktureinheit,
Sulfoxid = R₆-SO-R₇-, worin R⁶ und R⁷ unabhängig Alkyl oder Aryl sind,
Sulfonyl = R⁸-SO₂-R⁹, worin R⁸ und R⁹ unabhängig Alkyl oder C₆-C₁₂-Aryl sind,
Sulfid = R⁸-S-R⁹, worin R⁸ und R⁹ wie vorstehend sind,
dadurch gekennzeichnet, daß die Reaktion unter Verwenden von Ethylenglykol, Glycerin oder deren Gemischen als Lösungsmittel ausgeführt wird.

2. Verfahren von Anspruch 1, bei welchem in dem Aminoalkohol (IX) R¹ und R² unabhängig Wasserstoff, Sulfid, Sulfoxid, Sulfonyl, Aryl oder substituiertes Aryl darstellen und R³ und R⁴ unabhängig Wasserstoff oder -CH₂OH darstellen.

3. Verfahren der Ansprüche 1 oder 2, bei welchem R¹ und R² unabhängig Wasserstoff oder 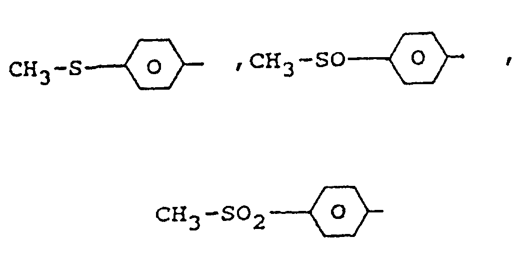 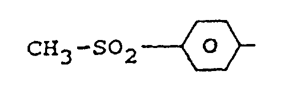 darstellen und R³ und R⁴ unabhängig Wasserstoff oder -CH₂OH darstellen.

4. Verfahren der Ansprüche 1, 2 oder 3, bei welchem in der Cyanverbindung (VIII) R⁵ Phenyl, 4-Nitrophenyl, Cinnamyl, 4-Methoxcinnamyl oder Dichlormethyl ist.

5. Verfahren der Ansprüche 1, 2, 3 oder 4, bei welchem die Base Diazabicycloundecen oder Kaliumcarbonat ist.

6. Verfahren der Ansprüche 1, 2, 3, 4 oder 5, bei welchem die Base in einer Menge eingesetzt wird, die von etwa 10 bis 0,01 Mol Base auf ein Mol Aminoalkohol reicht.

7. Verfahren der Ansprüche 1, 2, 3, 4, 5 oder 6, bei welchem das Alkohollösungsmittel Ethylenglykol ist.

8. Verfahren der Ansprüche 1, 2, 3, 4, 5, 6 oder 7, bei welchem das Alkohollösungsmittel ein Gemisch aus Ethylenglykol und Glycerin ist.

9. Verfahren der Ansprüche 1, 2, 3, 4, 5, 6, 7 oder 8, bei welchem das Zusammenbringen bei von etwa 70 bis etwa 150°C reichenden Temperaturen ausgeführt wird.

10. Verfahren der Ansprüche 1, 2, 3, 4, 5, 6, 7 oder 8, bei welchem das Zusammenbringen bei von etwa 100 bis etwa 110°C reichenden Temperaturen ausgeführt wird.

## Revendications

1. Procédé pour préparer un composé oxazoline de formule 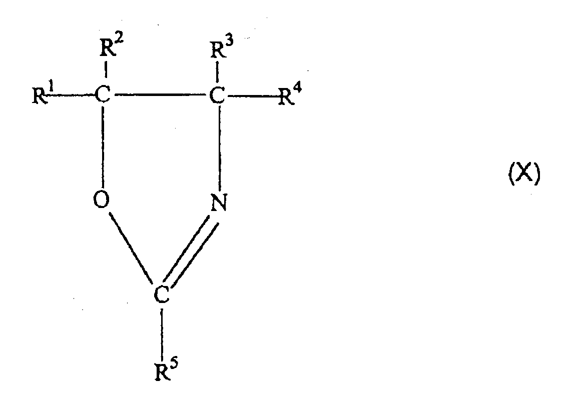 comprenant la mise en contact d'un composé cyano de formule
R⁵ - C = N (VIII)
avec un composé amino alcool de formule 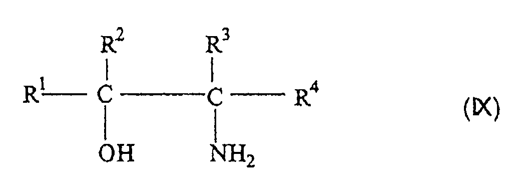 ou un sel de celui-ci, en présence d'une base,
où R¹, R², R³, R⁴, et R⁵ peuvent être identiques ou différents, à condition que R⁵ ne contienne pas de groupes cyano ou carboxyle, et que chacun indépendamment représente :
(a) un hydrogène, alkyle, alcynyle, alcynylakyle, hydroxyalkyle, haloalkyle, dialkylaminoalkye,
(b) un cycloalkyle, cycloalcalkyle, alkyle hétérocyclique, cycloakyle hétérocyclique, cycloalcalkyle hétérocyclique, alcényle, alcénylalkyle, alcoxyalkyle, aryle, arylalkyle, arylalcényle, alcoxyaryle, aryloxyaryle, aryloxyalkyle, acyle, aromatique hétérocyclique, sulfoxyde, sulfonyle, sulfure, ou
(c) les moitiés listées en (b) substituées par un halo, alkyle, aryle, cyano, carboxyle ou les sels de ceux-ci, nitro ou hydroxyle,
où alkyle = alkyle en C₁-C₆
aryle = aryle en C₆-C₁₅,
acyle = une moitié carbonyle liée à un hydrogène, un alkyle, un aryle, un alcoxy, un amino ou un aryloxy,
sulfoxyde = R₆-SO-R₇- où R₆ et R₇ sont indépendamment un alkyle ou un aryle,
sulfonyle = R⁸-SO₂-R⁹, où R⁸ et R⁹ sont indépendamment un alkyle ou un aryle en C₆-C₁₂,
sulfure = R⁸-S-R⁹, où R⁸ et R⁹ sont tels que ci-dessus;
caractérisé en ce que la réaction est effectuée en utilisant, comme solvant, de l'éthylène glycol, du glycérol ou des mélanges de ceux-ci.

2. Procédé selon la revendication 1 dans lequel en ce qui concerne l'amino alcool (IX), R¹ et R² indépendamment représentent un hydrogène, un sulfure, un sulfoxyde, un sulfonyle, un aryle ou un aryle substitué; et R³ et R⁴ représentent indépendamment un hydrogène ou -CH₂OH.

3. Procédé selon la revendication 1 ou 2 dans lequel R¹ et R² représentent indépendamment un hydrogène ou 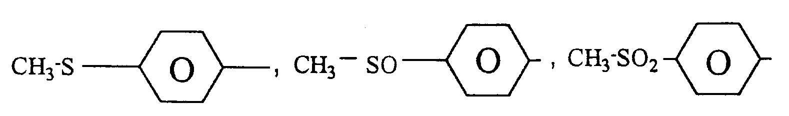 et R₃ et R₄ indépendamment représentent un hydrogène ou -CH₂0H.

4. Procédé selon les revendications 1, 2 ou 3 dans lequel en ce qui concerne le composé cyano (VIII) R⁵ est un phényle, un 4-nitrophényle, un cinnamyle, un 4-méthoxycinnamyle ou un dichlorométhyle.

5. Procédé selon les revendications 1, 2, 3 ou 4 dans lequel la base est du diazabicycloundécène ou du carbonate de potassium.

6. Procédé selon les revendications 1, 2, 3, 4 ou 5 dans lequel la base est employée en une quantité dans l'intervalle d'environ 10 à 0,01 mole de base pour une mole d'amino alcool.

7. Procédé selon les revendications 1, 2, 3, 4, 5 ou 6 dans lequel le solvant alcool est l'éthylène glycol.

8. Procédé selon les revendications 1, 2, 3, 4, 5, 6 ou 7 dans lequel le solvant alcool est un mélange d'éthylène glycol et de glycérol.

9. Procédé selon les revendications 1, 2, 3, 4, 5, 6, 7 ou 8 dans lequel la mise en contact est effectuée à des températures dans l'intervalle d'environ 70 à environ 150°C.

10. Procédé selon les revendications 1, 2, 3, 4, 5, 6, 7 ou 8 dans lequel la mise en contact est effectuée à des températures dans l'intervalle d'environ 100 à environ 110°C.
